# EUROPEAN PATENT SPECIFICATION

(11) **EP 2 547 304 B1**
(45) Date of publication and mention of the grant of the patent: **25.05.2016**
(21) Application number: 11731151.4
(22) Date of filing: 14.03.2011
(51) Int. Cl.: A61F 13/26

(54) **APPLICATOR FOR EJECTING OF CONTENTS, PREFERABLY TAMPONS**
APPLIKATOR ZUR AUSGABE VON GEGENSTÄNDEN, INSBESONDERE TAMPONS
APPLICATEUR PERMETTANT D'ÉJECTER LE CONTENU, DE PRÉFÉRENCE DES TAMPONS

(30) Priority: 14.03.2010 SI 201000088
(43) Date of publication of application: 23.01.2013
(73) Proprietor: TOSAMA Tovarna sanitetnega materiala d.o.o., 1230 Domzale (SI)
(72) Inventor: STRAZAR, Sandi, 1230 Domzale (SI)
(74) Representative: Borstar, Dusan
(86) International application number: PCT/SI2011/000013
(87) International publication number: WO 2011/115586

(56) References cited:
- WO-A1-2010/046478
- US-A- 4 361 150
- US-A- 4 479 791
- US-A- 5 554 109
- US-A1- 2008 195 029

## Description

Technical problem is size of the applicators before ejecting of the contents inside the tubes, preferably before ejecting the tampon inside the vagina of the user as well as sliding of the inner piston in form of the tube along the content which is subject of the ejection in desired direction.

There are several applicators on the market all used for the same purposes i.e. to help the user during insertion of tampons inside the vagina.

Patent US 5,709,652 (Hagerty) shows the applicator with inner piston used for ejection of the tampon into the vagina of the user. The patent does not explain construction of the piston, in Fig. 6 said patent is only shown flat pushing surface of the piston.

The solution in which the piston has design of the tube is summarized in patent application WO 20100243398 /Watanabe Hitoshi and Kikuchi Akia), published on March 4, 2010. The solution however does not solve the technical problem as presented herein as the tampon before the use is in the outer tube resulting in applicator which is not compact enough.

A hygienic applicator is disclosed in US 4,361,150 (Voss), which consists of an outer tube and an inner tube i.e. a so-called plunger, which is coaxially i.e. telescopically inserted within said outer tube.

Moreover, a tampon applicator is disclosed in WO2010/046478 A1 cited on Art 54(3) EPC, which consists of an outer tube and an inner tube i.e. a so-called plunger, which is co-axially i.e. telescopically inserted within said outer tube.

Tampons are also disclosed in US 4 479 791 A, US 5 554 109 A and US 2008/195029 A1. The above referenced technical problem is solved by applicator for ejecting contents as in Claim 1, preferably tampons where the essence of the invention is described below. The essence of the invention is in construction of the inner tube where the end is constructed in such a way to form one way (non-return) means in this embodiment one-way stop device, preferably a plurality of flaps bent at angle 30° from the longitudinal axis.

In particular embodiment the applicator is used for insertion of tampons into vagina of the user, but can also be used for insertion of medicaments into the position of the application or ejecting of technical devices into flexible or fixed tubes.

The applicator comprises at least two co-axial hollow tubes. Before the use in the most inner tube of co-axial tubes there is contents which is to be ejected with applicator. The applicator is used by pulling out the inner tube in such fashion that the contents remains in the outer tube (if two co-axial tubes are used) or next tube counting from inside toward outside. This solution solves the technical problem of compact size as the piston does not have to be appended separately or does not project outward from the tube with the tampon.

As the inner tube is embodied as one-way stop device after extraction (pulling out) of the inner tube the inner tube cannot be re-inserted in initial position without at the same time inner tube itself (by virtue of the shape) or with it (by virtue of friction or other force exerted onto the contents) push the contents and in preferred embodiment a tampon. The inner tube acts as a piston which is known as such in state of the art but not in such construction as presented herein.

Preferably the one-way stop means, preferably one-way stop device is designed in such a manner that the end of the inner tube is formed into flaps. Preferably the inner tube is made of plastic material by injection molding so the flaps can be made (bent) in desired angle relative to the longitudinal axis.

The test have shown that in cases of flaps being bent from the longitudinal axis for less than 10° the one-way stop device does not work properly. The flaps are elastic and in case of pushing of the inner tube into the initial position they slide along the contents, on particular embodiment along the tampon. In such case the content can no longer be pushed neither by form nor by force in desired direction.

The test have shown that the best effects are achieved with angle of the flaps 30°.

Below the essence of the invention is shown with help of figures whereas the figures form integral part of this patent application, and show:

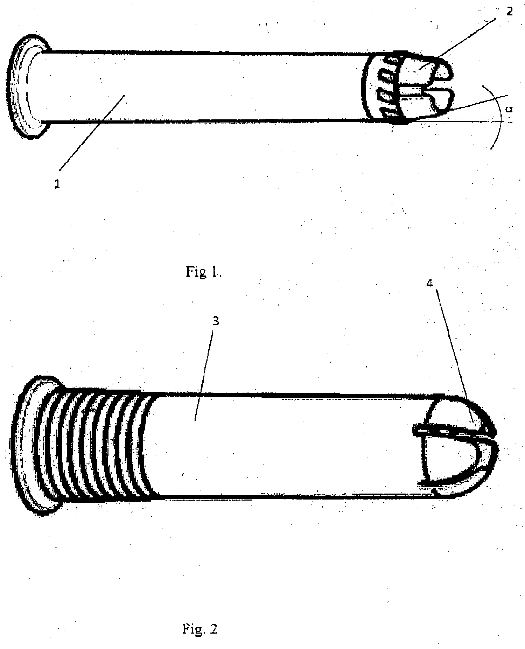

Figure 1 shows inner tube 1, flaps 2 and angle a of flaps 2 a from the longitudinal axis.
Figure 2 shows outer tube 3 as follows, outer tube 3, opening 4.

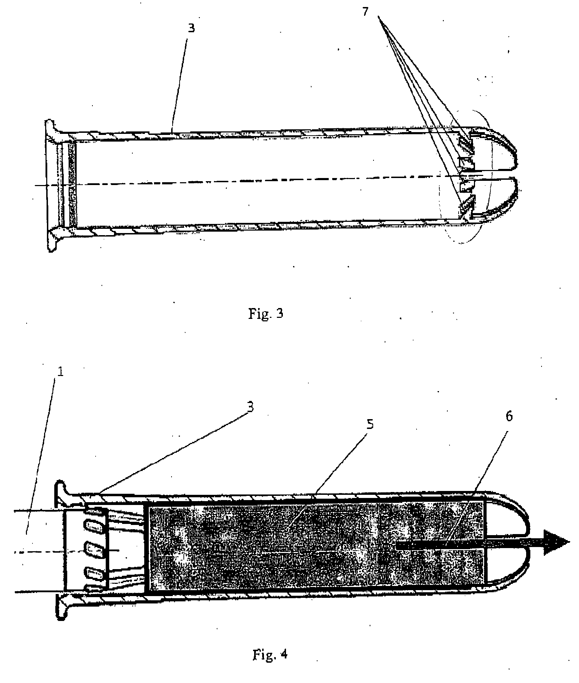

Figure 3 shows the cross-section of outer tube 3 as follows: outer tube 3 and teeth 7 for holding the contents 5.
Figure 4 shows the applicator during the ejection: inner tube 1, outer tube 3, contents 5, preferably tampons, and direction 6 of ejection.

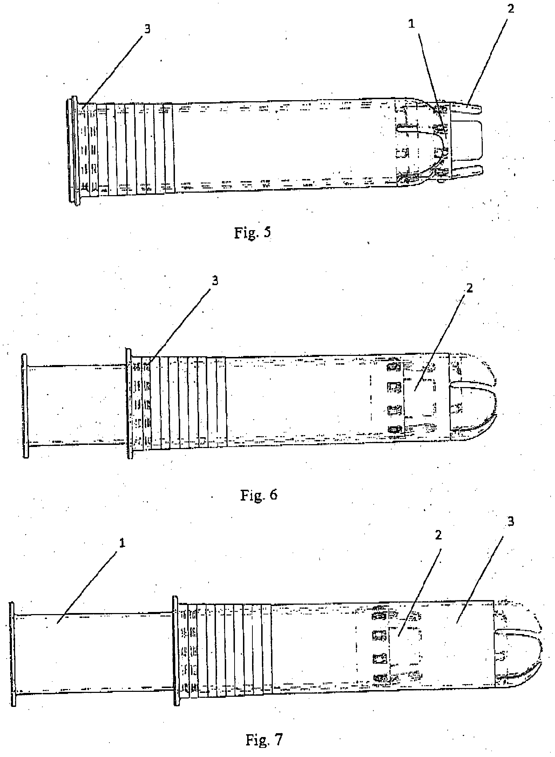

Figure 5 shows inner tube 1, outer tube 3 and flaps 2 in closed position, before pulling-out.
Figure 6 shows inner tube 1, outer tube 3 and flaps in closed position, during pulling-out.
Figure 7 shows inner tube 1, outer tube 3 and flaps 2 in closed position, after pulling out.

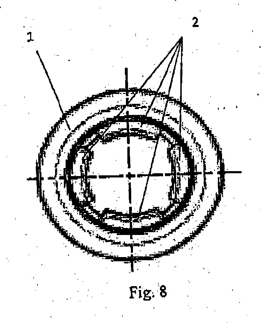

Figure 8 shows cross-section of inner tube 1 and flaps 2 in cross-section.

Below the preferred embodiment of tampon will be described with help of figures even though the invention can be used as already stated, for other contents such as medications or machine elements to be inserted in flexible or rigid tubes.

The one-way stop device according to the embodiment comprises the flaps 2 along the circumference, said flaps are bent inward in such fashion that middle of tips of the flaps 2 form virtual essentially circle with diameter smaller than the diameter of the inner tube 1. The flaps 2 are elastic and can be moved away from or toward the longitudinal axis of the invention. According to this invention the flaps slide along contents 5 (tampon)so long that the contents 5 is extracted (pulled-out), then they due to their elasticity move toward (close) and do not let the contents 5 (tampon) to move in the opposite direction so they form one-way stop device. The user then pushes inner tube 1 in direction 6 where contents is to be pushed out from the outer tube, and inner tube 1 acts as piston with flaps 2 as top of said piston.

Before the use of the contents 5 (tampon) is secure inside the inner tube 1 preferably in such fashion that contents 5 (tampon) at least partially lean against inner wall of outer tube 3. The flaps 2 of inner tube 1 are separated and are essentially parallel to longitudinal axis.

During use the user extracts (pulls-out) the inner tube 1 in opposite direction of desired direction of insertion (ejection). The flaps 2 allow this as they are flexible (elastic) enough to separate and allow movement of contents 5 (tampons) relative to inner tube 1. The contents 5 (tampon) remains in outer tube 3 which can be achieved by appropriate manufacturing of outer wall of inner tube 1 which should essentially smooth and inner wall of outer tube 3, which should be appropriately rough so that the wall of the contents 5 (tampon) exerts force on inner wall of outer tube 3.

In preferred embodiment the teeth 7 for holding the contents hold the contents 5 not to move in the same direction with inner tube 1, however this can also be achieved by some other means. The teeth 7 hold the contents 5 between the flaps 2 of inner tube 1 in such a fashion that inner tube can be longer than contents 5. In this case the contents 5 is stopped during pulling out of inner tube 1, the flaps of inner tube are separated so that the contents 5 can slide relative to the inner tube while inner tube 1 continues to pull out while the contents does not move relative to outer tube 3.

The contents 5 (tampon) is usually elastic and is pressed inside inner tube 1, and expands during pulling out of inner tube 1 to fill the outer tube 3.

When the inner tube is pulled out enough so that the whole contents 5 (tampon) is in outer tube 3 the flaps 2 of inner tube are set in position they were in before the contents 5 (tampon) was inserted in inner tube 5 i.e. they are bent relative to the longitudinal axis for angle a, which is 30°.

When the user starts to push inner tube 1 in desired direction 6. The flaps are now bent relative to longitudinal axis and do not let contents 5 (tampon) slide in inner tube 1. The inner tube therefore acts as piston and ejects contents 5 (tampon) in desired direction

There the surprising technical effect can be seen. If the flaps 2 would be bent for 10° or less they would yield during pushing in desired direction 6 or would separate enough for contents 5 (tampon) to slide again inside inner tube 1. In such case the ejection would end and the invention would not have achieved its goal, i.e. ejection of contents, preferably tampon in desired direction.

## Claims

1. Applicator for ejecting of contents, preferably tampon, comprising two co-axial tubes, namely an inner tube (1) with one way stop means, an outer tube (3) without any supporting means, and contents (5) for ejecting, which is before use comprised within said inner tube (1), wherein the one way stop means comprise along the circumference of the inner tube (1) positioned, preferably essentially equidistant flaps (2), said flaps (2) form a virtual truncated cone and are bent inward, if not under load, for angle (a), **characterized in that** said angle (a) is 30° toward longitudinal axis of the applicator, by which the middle of tips of the flaps (2) form essentially virtual circle with diameter smaller than the diameter of the inner tube (1).

## Patentansprüche

1. Applikator zur Ausgabe von Inhalt, vorzugsweise Tampons, weicher zwei koaxiale Rohre aufweist, d. h. ein Innenrohr (1) mit einseitig gerichteten Anschlagmitteln, ein Außenrohr (3) ohne ein Stützmittel, und Inhalt (5) zum Ausgeben, welcher vor Gebrauch im Innenrohr (1) enthalten ist, wobei die einseitig gerichteten Anschlagmitteln positioniert entlang des Umfangs des Innenrohrs (1) vorzugsweise im Wesentlichen im gleichen Abstand angeordnete Zungen (2) aufweisen, wobei die Zungen (2) einen virtuellen Kegelstumpf bilden und nach innen gebogen sind, wenn nicht unter Last, in einem Winkel (a), **dadurch gekennzeichnet, dass** der Winkel (a) 30° zur Längsachse des Applikators beträgt, wobei die Mitte der Spitzen der Zungen (2) einen im Wesentlichen virtuellen Kreis bilden, wobei der Durchmesser kleiner als der Durchmesser des Innenrohrs (1) ist.

## Revendications

1. Applicateur pour éjecter un contenu, préférablement un tampon, comprenant deux tubes coaxiaux, à savoir un tube intérieur (1) avec un moyen d'arrêt unidirectionnel, un tube extérieur (3) sans aucun moyen de support, et un contenu (5) pour éjection, qui est avant utilisation compris à l'intérieur dudit tube intérieur (1), dans lequel le moyen d'arrêt unidirectionnel comprend, le long de la circonférence du tube intérieur (1) positionné, des onglets (2) préférablement sensiblement équidistants, lesdits anglets (2) forment un cône tronqué virtuel et sont courbés vers l'intérieur, s'ils ne subissent pas de charge, à un angle (a), **caractérisé en ce que** ledit angle (a) est 30° vers un axe longitudinal de l'applicateur, le milieu des pointes des onglets (2) formant un cercle essentiellement virtuel avec un diamètre plus petit que le diamètre du tube intérieur (1),
